# EUROPEAN PATENT SPECIFICATION

(11) **EP 0 317 873 B1**
(45) Date of publication and mention of the grant of the patent: **12.05.1993**
(21) Application number: 88118990.6
(22) Date of filing: 14.11.1988
(51) Int. Cl.: C07D 207/09, A61K 31/40, C07B 59/00

(54) **Radioiodinated benzamides and method of their use as radioimaging agents**
Radiojodhaltige Benzamide und Verfahren zu ihrer Verwendung als radiobildende Agenzien
Benzamides radioiodées et méthode pour leur utilisation comme agents de radioimages

(30) Priority: 19.11.1987 US 122390
(43) Date of publication of application: 31.05.1989
(73) Proprietor: THE VANDERBILT UNIVERSITY, Nashville, TN 37203 (US)
(72) Inventor: De Paulis, Tomas, Nashville, TN 37204 (US); Kessler, Robert M., Nashville, TN 37215 (US); Smith, Howard E., Nashville, TN 37215 (US); Janowsky, Aaron, Portland, OR 97214 (US); Clanton, Jeffrey A., Nashville, TN 37221 (US)
(74) Representative: Grünecker, Kinkeldey, Stockmair & Schwanhäusser Anwaltssozietät

(56) References cited:
- US-A- 4 279 887
- CHEMICAL ABSTRACTS, vol. 109, no. 9, August 1988, pages 49, abstract no. 66715n, Columbus, Ohio, US; A. JANOWSKY et al.: "Iodopride: a specific high affinity radioligand for labeling striatal dopamine D-2 receptors"
- CHEMICAL ABSTRACTS, vol. 109, no. 7, 1988, page 328, abstract no. 50956f, Columbus, Ohio, US; T. BRUCKE et al.: "In vitro binding properties and autoradiographic imaging of 3-iodobenzamide ([125I]-IBZM): a potential imaging ligand for D-2 dopamine receptors in SPECT"
- JOURNAL OF MEDICINAL CHEMISTRY, vol. 31, no. 5, May 1988, pages 1039-1043, American Chemical Society, US; H.F. KUNG et al.: "Dopamine D-2 receptor imaging radiopharmaceuticals: Synthesis, radiolabeling, and in vitro binding of (R)-(+)- and (S)-(-)-3-Iodo-2-hydroxy-6-methoxy-N-[(1-ethyl-2-pyrrolidinyl)methyl]benzamide"
- JOURNAL OF MEDICINAL CHEMISTRY, vol. 31, no. 10, October 1988, pages 2027-2033, American Chemical Society, US; T. DE PAULIS et al.: "(S)-N-[(1-Ethyl-2-pyrrolidinyl)methyl]-5-[125I]iodo-2-methoxybenzamide hydrochloride, a new selectrive radioligand for dopamine D-2 receptors"
- CHEMICAL ABSTRACTS, vol. 108, no. 17, April 1988, page 362, abstract no. 146341g, Columbus, Ohio, US; H.F. KUNG et al.: "Preparation and biodistribution of [125I]IBZM: a potential CNS D-2 dopamine receptor imaging agent"
- CHEMICAL ABSTRACTS, vol. 108, no. 17, April 1988, pages 362, abstract no. 146292s, Columbus, Ohio, US; H.F. KUNG et al.: "Comparison of in vivo D-2 dopamine receptor binding of IBZM and NMSP in rat brain"
- CRAWLEY, F.C.W. et al., Clin. Sci. 70, Abstract 145 (1986)
- KUNG, H.F. et al., J. Label Comp. Radiopharm. 23, 1318 (1986)

## Description

The present invention relates to new radiolabeled compounds and a radiopharmaceutical composition comprising same. More specifically, the present invention relates to substituted benzamides which in radioiodinated form can be used in a radiopharmaceutical composition as an imaging agent, particularly for the brain.

Radiolabeled compounds which are subject to localization in particular organs or tumors therein are of great value for diagnosis of diseases of the human body. For example, thallium 201 and fatty acids labeled with carbon-11 and iodine-123 have been utilized as heart imaging agents. Various phosphonate ligands labeled with technetium-99m have been used to image infarcted regions of the heart. However, although many useful radiolabeled compounds are known, there remains a need for the discovery of improved compounds which are effective for routine imaging of the brain. In particular, there remains a need for radiolabeled compounds which are useful in imaging the dopamine D-2 receptor of the brain.

Butyrophenones and substituted benzamides labeled with iodine-125, iodine-123, and iodine-131 have been found to image dopamine receptors of the brain. However, previous agents lack the ability to selectively label only subpopulations of the dopamine D-2 receptors implicated in psychiatric disorders.

Japanese Patent Application 59112971 discloses an agent for detecting changes in dopamine receptors having the following structure: 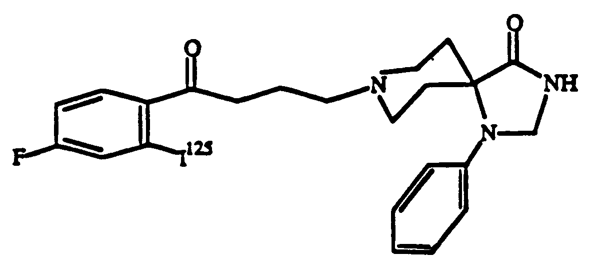
Landwater, S.W., Label Comp. Radiopharm. 22:273-278 (1985), disclosed the following compound which is described as a high affinity dopamine receptor probe: 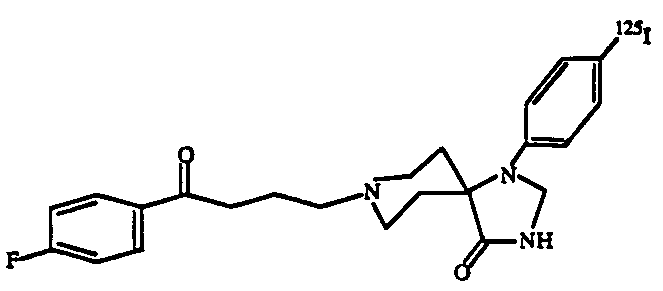
Crawley, F.C.W., et al., Clin. Sci. 70:Suppl. 13, Abstr. 145 (1986), and, independently, Kung, H.F., et al., J. Label Comp. Radiopharm. 23:1318-1319 (1986) discloses the following compound which is described as an agent for the studying of dopamine receptors in vivo, dopamine D-2 receptors in the brain, in particular. 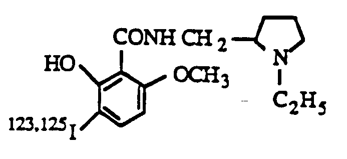
This compound, having a non-radioactive isotope of iodine, was disclosed in European Patent Application EP 60235.

However, no mention is made with respect to an improved binding selectivity to a hypothetical subpopulation of dopamine D-2 receptors in the brain thought to be involved in psychiatric disorders.

Further, radioidine containing compounds useful as brain imaging agents are disclosed in US Patent Application 4,279,887 and have the general formula 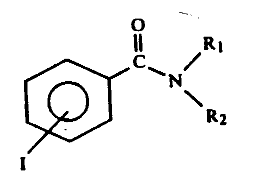
wherein I is a radioisotope of iodine with I-123 being preferred, R₁ and R₂ are the same or different and are selected from the group consisting of hydrogen, hydroxy, alkyl, aryl, substituted aryl, aralkyl, anilino and carbamoylmethyl or R₁ and R₂ taken together with the nitrogen to which they are attached form a 5- or 6-membered ring.

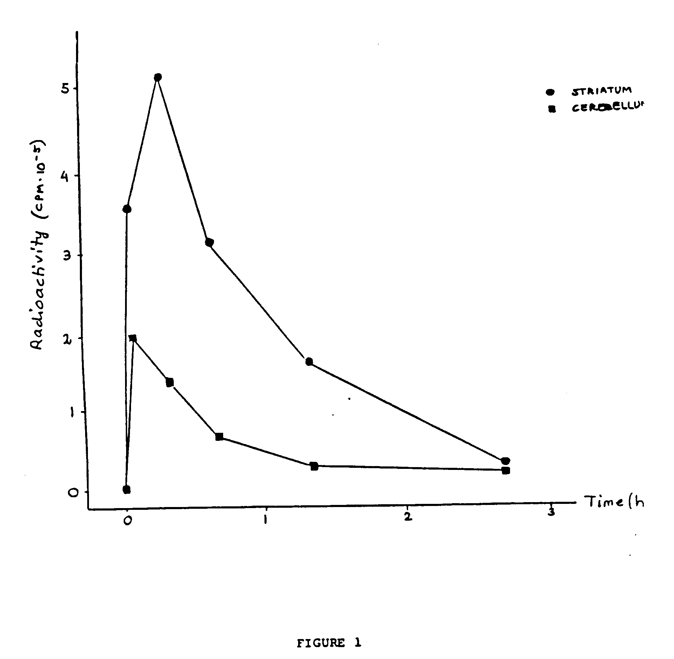

European Patent Application EP 207913 discloses the following compound which is described as a brain dopamine receptor blocker: 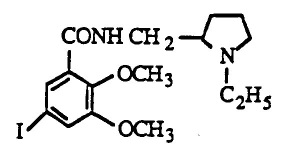
European Patent Application EP 156776 discloses the following compound which is described as a neuroleptic agent: 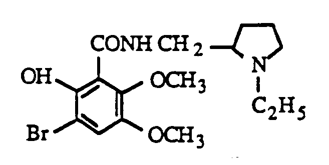
Neumeyer, J.L., et al., J. Med. Chem. 28:405-407 (1985), disclosed the following compound which is described to have receptor binding behaviour similar to that of haloperidol: 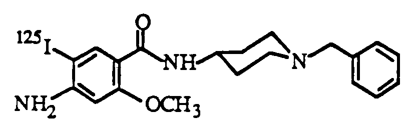
Wilson, A.A., et al., J. Nucl. Med. 28:729 (1987), disclosed the following compound which is described to bind to brain receptors blockable by haloperidol: 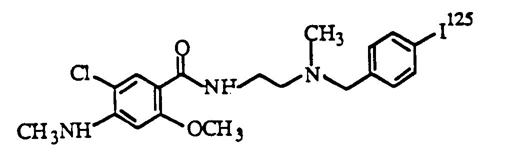
Martres, M.-P., et al., Eur. J. Pharmacol. 118:211-219 (1985), disclosed the following compound which is described as a selective ligand for dopamine D-2 receptors: 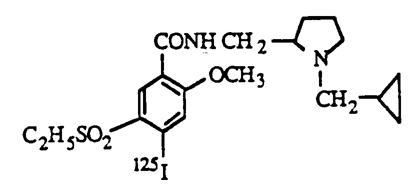
FIG. 1 is a graphic representation of in vivo binding studies of the radioiodinated substituted benzamine of the present invention demonstrating its properties of having rapid penetration into the rat brain and labeling of the dopamine D-2 receptor.

It is the object underlying the present invention to provide novel radiopharmaceutical substituted benzamides as well as radiopharmaceutical compositions comprising these compounds which compared with the compounds of the prior art have the advantage that a) their preparation and radioiodination is facile, b) both enantiomers can be used for enhanced image contrast, and c) they bind selectively to a hypothetical subpopulation of dopamine D-2 receptors in the brain thought to be involved in psychiatric disorders.

This object is obtained with a compound of general formula I 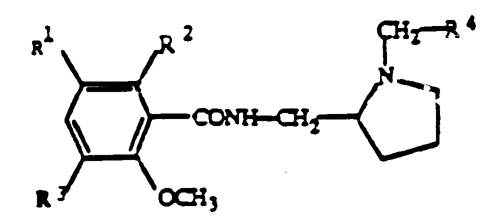
wherein R¹ is ¹²³I, ¹²⁵I, ¹³¹I, R² is a hydrogen atom or a hydroxyl group, R³ is a hydrogen atom, a halogen atom, a hydroxyl group, or a methoxy group, R⁴ is an alkyl group of 1 to 4 carbon atoms, an alkenyl group of 2 to 4 carbon atoms, an alkynyl group or 2 to 4 carbon atoms, cycloalkyl group of 3 to 7 carbon atoms, a phenyl group, a para-halogen-substituted phenyl group, or an enantiomer thereof, under the proviso that when R² is a hydroxyl group R³ is not a hydrogen atom.

A radiopharmaceutical composition of the present invention comprises an effective amount of at least one compound of the above general formula I and suitably a pharmaceutical carrier such as physiological buffered saline solution.

Various substituted benzamides which undergo the radioiodination reaction of the present invention are either known compounds or can be formulated by the following procedures. Briefly, the benzamides can be obtained by reacting a halogen substituted benzamide with bis(trialkyltin) in the presence of a palladium catalyst. For example, substituted benzamide derivatives represented by the formula: 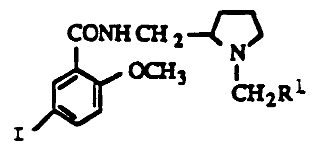
wherein R¹ is a hydrogen atom, a lower alkyl group consisting of 1 to 4 carbon atoms, a cycloalkyl group consisting of 3 to 7 carbon atoms, an alkenyl group consisting of 2 to 4 carbon atoms, an alkynyl group consisting of 2 to 4 carbon atoms, a phenyl group, a halogen-substituted phenyl group, or an enantiomer or pharmaceutically suitable salt thereof may be obtained by the reaction of the intermediate compound: 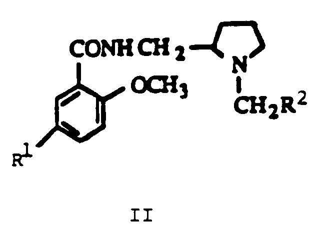
wherein R¹ is tributyltin group with a radioactive isotope of iodine in a protic solvent such as ethanol or hydrochloric acid. The iodine can be generated in situ by decomposition of iodine monochloride or by oxidation of the alkali metal iodide with hydrogen peroxide or an agent such as the sodium salt of N-chloro-p-toluenesulfonamide.

Further, compounds of the preferred formula can be obtained by reaction of the intermediate compound, wherein R¹ is a triazeno group of the formula: 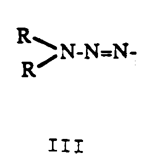
wherein R is a lower alkyl group of 1 to 4 carbon atoms or constitutes a 5- or 6-member ring, which is reacted with an acid, such as hydrochloric acid or trifluoroacetic acid, in the presence of iodide in the solvent, such as acetone, formic acid, or acetonitrile.

It has been found that when substituted benzamides are radioiodinated they are useful radiopharmaceuticals for imaging the dopamine D-2 receptor in the brain and therefore are useful in brain imaging experiments.

In vitro receptor binding studies of the radioiodinated substituted benzamides have shown them to be selective antagonists of central dopamine D-2 receptors. A compound of this invention (Example 1) could be competitively displaced to 50% at 1-2 nM inhibitory concentration by haloperidol or at 1.3 nM inhibitory concentration by raclopride from their binding sites in rat brain homogenates.

In vivo receptor binding studies of the radioiodinated substituted benzamides have shown a rapid penetration into the rat brain (Fig. 1). Peak uptake in the striatum was observed after 20 min. After 60 min, the ratio of radioactivity in the striatum was 7.3 times higher than that in the cerebellum, showing a preferential binding to dopamine-rich structures of the brain. Pretreatment of haloperidol reduced the uptake of the radioiodinated benzamide in striatum to 23% of that of untreated rats.

Radioiodinated substituted benzamide compounds suitable for use herein can be synthesized by the Sandmeyer reaction to introduce the radioligand. The intermediate diazonium derivative is generated by acid decomposition of the corresponding pyrrolidinotriaze. The benzamide compounds of this invention can also be obtained by acid-catalyzed iododestannylation of the corresponding trialkyltin derivatives. The radioiodination technique is illustrated in the examples. I-123, I-125, and I-131 radioligands are employed as imaging agents for the brain. A pharmaceutical composition of the present invention comprises one of the aforementioned isotopes of radioiodinated substituted benzamides and a carrier such as physiological buffered saline solution. When practically applied the composition will be systematically administered to the patient as by intravenous injection. Suitable dosages for use as a diagnostic imaging agent are from about 2 to about 20 mCi of I-123 labelled iodobenzamides for D-2 dopamine receptors, that is, as imaging agents for the brain. It will be appreciated by those skilled in the art that the novel imaging agent of the present invention is employed in accordance with conventional methodology in nuclear medicine in a manner analogous to functional brain imaging. Thus, a composition of the present invention can be systematically applied to the patient and subsequently the uptake of the composition in the selected organ is measured and an image formed, for example, by means of a conventional gamma radiation CT camera.

Further understanding and use of the present invention can be obtained from the examples and from Budinger, T.F., Physical Attributes of Single-Photon Tomography, J. Nucl. Med. 21:579-592 (1980).

In the process of preparing the radiolabelled substituted benzamides of the present invention, the radioactive iodine atom is introduced in the last step of the synthesis. In this manner, the radiation hazard will be limited in the preparation and purification of the ligands.

Preferred compounds of the present invention are: 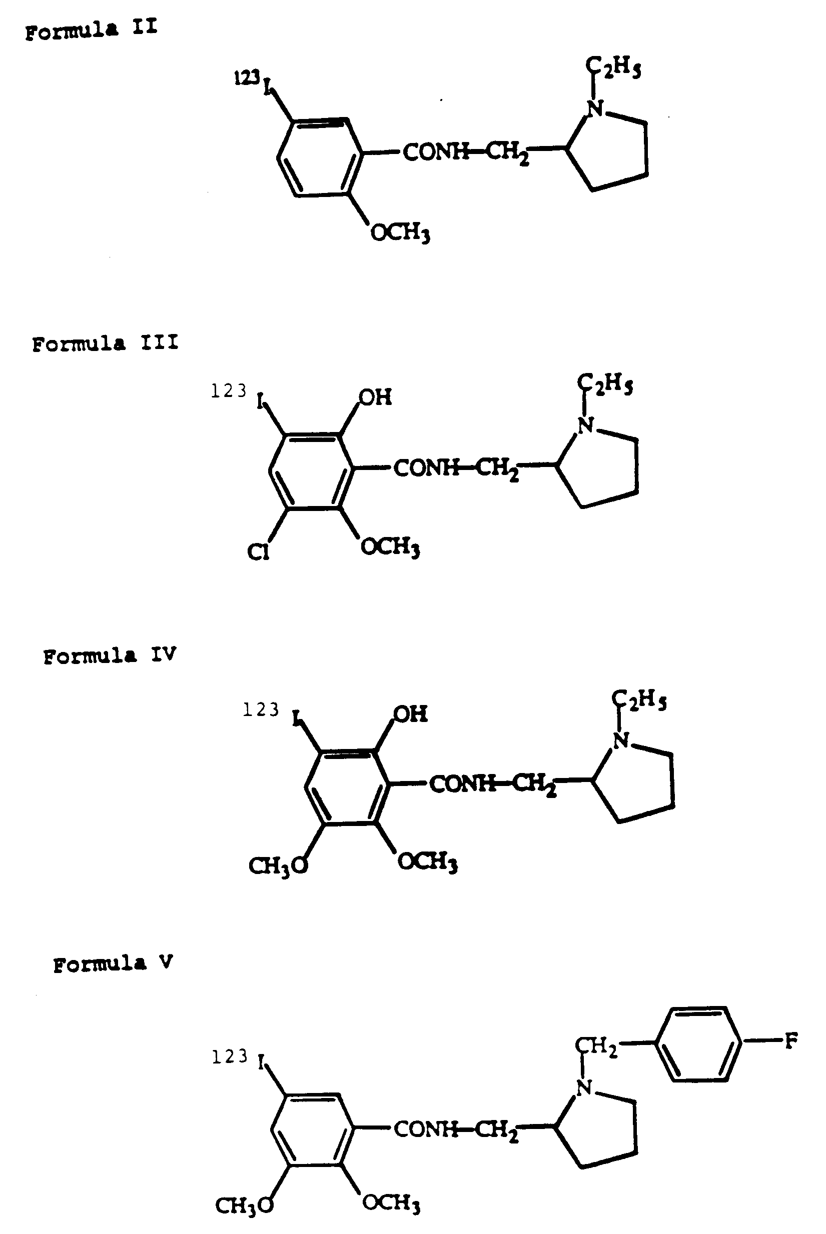
The above-noted compounds are used to detect, visualize and analyze the distribution and function of the dopamine D-2 receptor in the mammalian brain as racemic mixtures or as their optically resolved anantiomers. The corresponding enantiomers can be obtained by using the optical stereoisomers of the appropriate synthetic precursor.

Suitable iodine isotopes for single photon (gamma) auto radiography or computed tomography are iodine-123 for maximum specific radioactivity at 9 x 10⁶ Ci/mmol, iodine-125 for 2 x 10³ Ci/mmol, and iodine-131 for 1.6 x 10⁴ Ci/mmol.

Compounds of the present invention can be obtained by one of the following methods of synthesis. 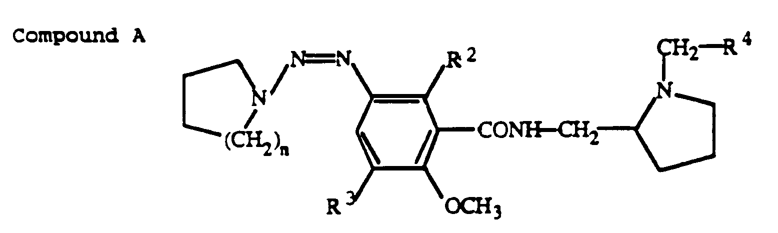
Compounds of Formula A wherein n = 1 or 2, R² is a hydrogen atom or a hydroxyl group, R³ is a hydrogen atom, a halogen atom, a hydroxyl or methoxyl group and R⁴ is an alkyl of 1 to 4 carbon atoms, an alkenyl group of 2 to 4 carbon atoms or an alkynyl group of 2 to 4 carbon atoms, a phenyl group, or a para-halogen-substituted phenyl group, are treated with an organic acid such as trifluoroacetic acid or an inorganic acid such as hydrochloric acid in the presence of radioactive sodium or potassium iodide in an aprotic solvent such as acetone, benzene, or acetonitrile. 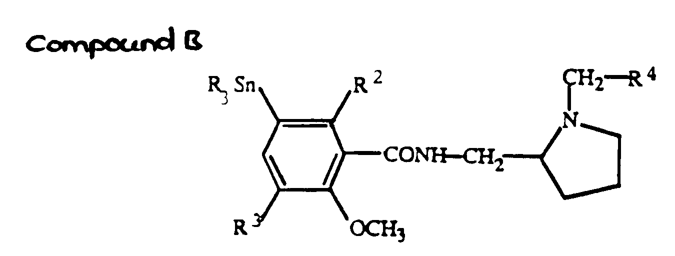
Compounds of Formula B wherein R is an alkyl of 1 to 5 carbon atoms such as n-butyl, R², R³ and R⁴ are defined as before are treated with radioactive iodine in a protic solvent such as ethanol or dilute hydrochloric acid. The iodine can be generated in situ by oxidation of sodium iodide with hydrogen peroxide or chloramine-T, or by using radioactive iodine monochloride. 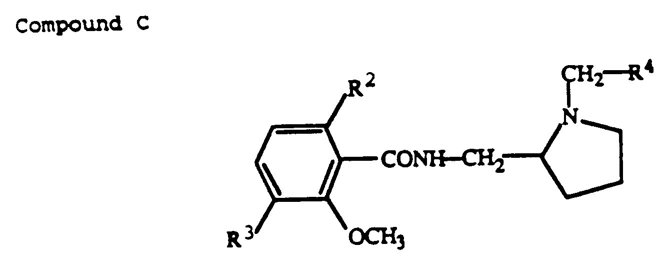
Compounds of Formula C wherein R², R³ and R⁴ are defined as in Formula I are treated with radioactive iodine in a solvent such as chloroform or dioxane at elevated temperature.

The following examples describe in detail compounds illustrative of the present invention and methods which have been devised for their preparation.

### EXAMPLE 1.

### [¹²⁵I](S)-N-[(1-Ethyl-2-pyrrolidinyl)methyl-5-iodo-2-methoxybenzamide.

A solution of (S)-N-[1-ethyl-2-pyrrolidinyl)methyl]-2-methoxy-5-[3,3-(1,4-butanediyl)]triazenebenzamide (1.5 µg, 4.7 nmol) in benzene (9 µl) was mixed with 1.8 mCi of Na¹²⁵I (0.5 µg, 520 nmol) in formic acid (10 µl) was added and the reaction mixture was shaken for 40 min at 20°C. Aqueous 1 N NaOH (300 µl) was added. Extraction with benzene (2 x 100 µl) gave the iodine substituted benzamide. Extraction of the combined organic layer with 0.1 N HCl (2 x 150 µl) gave 0.41 mCi of pure product. Specific activity 560 Ci/mmol, radiochemical yield 22%.

### EXAMPLE 2.

### [¹²⁵I](R)-N-[(1-Ethyl-2-pyrrolidinyl)-5-iodo-2-methoxybenzamide.

A solution of (R)-N-[(1-ethyl-2-pyrrolidinyl)methyl[-2-methoxy-5-tri-n-butyltinbenzamide (15 µg, 22 nmol) in diethylether (10 µL) was mixed with 10.4 mCi of Na¹²⁵I (3.2 µg, 22 nmol) in 0.001 N NaOH (29 µL). Hydrochloric acid (0.1 N, 10 µL) was added followed by the addition of an aqueous solution (5 µL) of sodium N-chloro-4-methylbenzene sulfonamide (16 µg, 70 nmol). After 10 min at 20°C, NaOH (2 N, 20 µL) was added. Extraction with ether (2 x 150 µL) gave 7.2 mCi of the desired iodobenzamide. Specific activity 590 Ci/mmol, radiochemical yield 70%.

### EXAMPLE 3.

### [¹²⁵I](S)-5-Chloro-N-[1-ethyl-2-pyrrolidinyl)methyl]-3-iodo-6-methoxysalicylamide.

A solution of (S)-5-chloro-N-[(1-ethyl-2-pyrrolidinyl) methyl]-6-methoxysalicylamide (de Paulis, T., et al., J. Med. Chem., 28:1263-1269 (1985)(9.4 µg, 30 nmol) in ethanol (30 µL) was mixed with a solution of 10 mCi of Na¹²⁵I (4.5 µg, 330 Ci/mmol, 30 nmol) in 0.001 N NaOH (30 µL). A solution of chloramine-T (13 µg, 50 nmol) in water (10 µL) was added. The mixture was heated to 60°C for 45 min. Addition of 0.1 N NH₄Cl (100 µL) and extraction with ether (2 x 150 mL) gave 7.6 mCi of product. Extraction of the combined organic layer with 0.1 N HCl (3 x 100 µl) gave 4.6 mCi of pure iodine substituted benzamide (specific activity 330 Ci/mmol). Thin layer chromatography (SiO₂, Merck F₂₅₄) in isopropylethermethanol-conc. NH₄OH (160:39:1) showed radioactivity at Rf 0.16, identical to that of an authentic sample. (The starting material had Rf 0.26). Radiochemical yield 46%.

### EXAMPLE 4.

### [¹²⁵I](S)-N-[1-Ethyl-2-pyrrolidinyl)methyl-3-iodo-5,6-dimethoxysalicylamide.

A solution of (S)-N-[(1-ethyl-2-pyrrolidinyl)methyl]-5,6-dimethoxysalicylamide (Eur. Pat. Appl. EP 156 776)(6.8 µg, 22 nmol) in ethanol (20 µL) was added to 6.13 mCi of Na¹²⁵I (3.2 µg, 22 nmol) in 0.001 N NaOH (20 µL). A solution of chloramine-T (10 µg, 44 nmol) in water (7 µl) was added at 20°C. After 15 min 1N NH₄Cl (50 µL) and 2 N NH₄OH (20 µL) were added. Extraction with ether (3 x 150 µL) combining of the organic layer and extracting with 0.1 N HCl (2 x 100 µl) gave pure iodobenzamide (specific activity 220 Ci/mmol and radiochemical yield 72%). Thin layer chromatography showed radioactivity at Rf 0.36, identical to that of an authentic sample. The starting material had Rf 0.42.

### EXAMPLE 5.

### [¹²⁵I](R)-N-[(1(4-Fluorobenzyl)-2-pyrrolidinyl)methyl]-5-iodo-2-methoxybenzamide

A solution of (R)-N-[(1-(4-fluorobenzyl)-2-pyrrolidinyl) methyl]-5-iodo-2-methoxybenzamide hydrochloride hydrate (0.52 g, 1.0 mmol) in triethylamine (30 ml) was treated with palladium(II) acetate (20 mg, 0.10 mm0l), tetrakis(triphenylphosphine) palladium(0) (60 mg, 0.05 mmol), and bis(tributyltin)(0.61 g, 1.05 mmol) at 85°C for 3 h. Filtration and washing of the isoluble material with triethylamine (10 mL), followed by evaporation of the solvent gave 1.1 g of a yellow oil. Separation on silica gel with a gradient of hexane-isopropylether-ethyl acetate-ethanol gave fractions with the corresponding tributyltinbenzamide as an oil. A 2 N solution of this oil in ether was preapred. 1 mCi of Na¹²⁵I (specific activity 200 Ci/mmol, 45 nmol) was prepared in 0.1 N HCl (10 µL, 1 µmol) and added to the benzamide solution (25 µL, 50 nmol). An aqueous solution of chloramine-T (10 µL, 149 nmol) was added. After 30 min the reaction mixture was neutralized with an excess of 2N NaOH (20 µL, 40 µmol) and the product was extracted with ether (3 x 150 µL). TLC showed radioactivity at Rf 0.57 identical to that of the starting iodobenzamide.

### EXAMPLE 6 (Intermediates)

### (S)-(-)-N-[(1-Ethyl-2-pyrrolidinyl)methyl]-5,6-dimethoxy-3-tributyltinsalicylamide.

To a solution of (S)-(-)-N-[(1-ethyl-2-pyrrolidinyl) methyl]-2-bromo-5,6-dimethoxysalicylamide (GB 2153354) (0.42 g, 1.0 mmol) in triethylamine (30 mL, freshly distilled from calcium hydride) was added palladium(II) acetate (20 mg, 0.1 mmol), tetrakis(triphenylphosphine)palladium(0), (60 mg, 0.05 mmol) and bis(tributyltin) 0.85 g (1.4 mmol) under nitrogen atmosphere at 25°C. The mixture was heated to reflux (85°C) for 16 h. The solvent was evaporated and the residue was subjected to column chromatography on silica el (Merck, 0.063 - 0.200 mm) with hexeneethyl acetate (10:1) as eluent. Fractions with a single spot on TLC with Rf 0.58 in isopropylethermethanol-conc. ammonium hydroxide (160:39:1) were collected and the solvent was removed by evaporation. This gave 0.30 g (50%) of an oil. NMR of low field regions: δ 7.08 (s, 1H), 3.93 (s, 3H) and 3.84 ppm (s, 3H). Part of the butyl signal appears at 0.89 ppm.

By the same method, the following organotin-benzamides were prepared from their corresponding bromo or iodo derivatives:
(S)-(-)-N-[(1-Ethyl-2-pyrrolidinyl)methyl]-2-methoxy-5-tributyltinbenzamide TLC: Rf 0.32 (i-Pr₂O-MeOH-NH₃, 160:39:1) from the iodo-derivative (Rf 0.12) NMR: δ 8.26 ppm (d, J = 3 Hz, C(6)-H).
(S)-(-)-N-[(1-Ethyl-2-pyrrolidinyl)methyl]-6-methoxy-3-tributyltinsalicylamide. TLC: Rf 0.43 (i-Pr₂O-MeOH-NH₃, 160:39:1), from the bromo derivative (Rf 0.35), NMR: δ 7.33 ppm (d, J = 10 Hz, C(4)H).
(R)-(+)-N-[(1-(4-Fluorobenzyl)-2-pyrrolidinyl)methyl]-2,3-methoxy-5-tributyltinbenzamide, TLC: Rf 0.61 (i-Pr₂O-MeOH-NH₃, 160:39:1), from the iodo derivative (Rf 0.58), NMR δ 8.32 ppm (d, J = 2.6 Hz, C(6)-H)
(S)-(-)-N-[(1-Ethyl-2-pyrrolidinyl)methyl]-2,3-dimethoxy-3-tributyltinbenzamide. TLC: Rf 0.56 (i-Pr₂O-MeOH-NH₃, 160:39:1), from the iodo derivative (Rf 0.53), NMR δ 7.77 (d, J = 1.2 Hz) and 7.11 ppm (d, C(4)-H).

### EXAMPLE 7.

Sulpiride, a neuroleptic agent, has a high selectivity in blocking the D-2 receptor, see Fuxe, K. et al., Neurosci. Lett. 64:163-168 (1986). Its radioligand [³H]sulpiride, was compared to [¹²⁵I](S)-N-[(1-ethyl-2-pyrrolidinyl)methyl]-5-iodo-2-methoxybenzamide ([¹²⁵I](S)-II in a receptor binding assay.

Male Sprague-Dawley rats (150-200 g) were killed by decapitation and their brains quickly removed and dissected on ice. Striata were dissected and homogenized in 40 vol of ice-cold Tris-HCl (50 mM) containing 123 mM NaCl, 5 mM KCl, 2 mM CaCl₂ and 1 mM MgCl₂ (pH 7.4) using a Brinkman Polytron. The suspension was centrifuged at 30,000 x g for 10 min at 4°C, and the pellet was resuspended in 40 vol of fresh buffer with polytron. The suspension was incubated at 37°C for 10 min, centrifuged again at 30,000 x g for 10 min (4°C) and the final pellet was resuspended with the polytron in 200 vol of ice cold buffer. Assay tubes contained 50 µM or radioligand at 2.9 nM concentration and 90 µL of the striatal membrane preparation. Nonspecific binding was determined in the presence of 10 µM(-)[¹²⁵I](S)-II. Tubes were incubated for 30 min at 37°C, poured over Whatman GF/B filters under vacuum, and the filters were rinsed three times with 4 mL of ice cold buffer. Radioactivity remaining on the filters was measured by liquid scintillation on a Quantum 9 Multichannel analyzer operating at 35% efficiency. Results are shown in Table I.

**TABLE I**

| Compound^{a} | [¹²⁵I]-(S) II binding^{b} IC₅₀(nM) | [³H]sulpiride^{c} binding IC₅₀(nM) |
|---|---|---|
| (R)-II | 169 | 146 |
| (S)-II | 5.0 | 3.6 |
| Sulpiride | 45 | 28 |

| | | |
|---|---|---|
| a) Each compound was dissolved in 0.2mL of HOAc and diluted with buffer. | | |
| b) Radioligand concentration 0.46 nM. Nonspecific binding was determined in the presence of 10 µM of (S)-II. | | |
| c) Radioligand concentration 2.9 nM. Nonspecific binding was determined in the presence of 10 µM sulpiride. | | |

The binding of [¹²⁵I](S)-II to rat striated membranes was saturable, reversible and stereospecific under the conditions described above. Binding reached equilibrium at 37°C within 20 min, and remained stable for at least 60 min. Scatchard anlysis of the saturation isotherm revealed a single set of binding sites with a K_{d} of 1.0 nM and a maximal number of binding sites of 169 fmol/mg protein. Sulpiride displaced the radioligand with an IC₅₀ of approximately 45 nM. In a displacement experiment using [³H](S)-(-)-sulpiride as the radioligand, the two enantiomers (R)-II and (S)-II had IC₅₀ values of 146 nM and 3.6 nM, respectively. (RS)-sulpiride had IC₅₀ 28 nM.

## Claims

1. A compound of general formula I 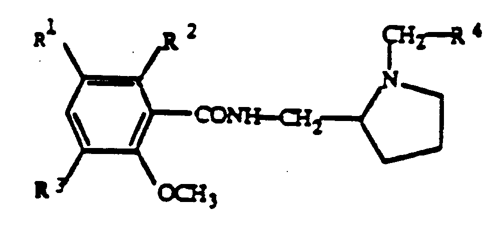 wherein R¹ is ¹²³I, ¹²⁵I, ¹³¹I, R² is a hydrogen atom or a hydroxyl group, R³ is a hydrogen atom, a halogen atom, a hydroxyl group, or a methoxy group, R⁴ is an alkyl group of 1 to 4 carbon atoms, an alkenyl group of 2 to 4 carbon atoms, an alkynyl group or 2 to 4 carbon atoms, cycloalkyl group of 3 to 7 carbon atoms, a phenyl group, a para-halogen-substituted phenyl group, or an enantiomer thereof, under the proviso that when R² is a hydroxyl group R³ is not a hydrogen atom.

2. The compound of claim 1 wherein R¹ is an isotope selected from I-123, I-125 and I-131.

3. The compound of claim 1 wherein R¹ of the compound is the I-123 isotope.

4. The compound of claim 1 wherein R¹ of the compound is the I-125 isotope.

5. The compound of claim 1 wherein R¹ of the compound is the I-131 isotope.

6. [¹²³I] (S)-N- [(1-ethyl-2-pyrrolidinylmethy]-5-iodo-2-methoxybenzamide.

7. [¹²³I] (R)-N- [(1-(4-fluorobenzyl)-2-pyrrolidinyl) methyl]-5-iodo-2-methoxybenzamide.

8. [¹²³I] (S)-N- [(1-ethyl-2-pyrrolidinyl)methyl]-5-chloro-3-iodo-6-methoxysalicylamide.

9. [¹²³I](R)-N- [(1-(4-fluorobenzyl)-2-pyrrolidinyl) methyl]5-iodo-2,3-dimethoxybenzamide.

10. [¹²³I] (S)-N-[(1-ethyl-2-pyrrolidinyl)methyl] -5-iodo-2,3-dimethoxybenzamide.

11. [¹²³I] (S)-N-[(1-ethyl-2-pyrrolidinyl)methyl] -3-iodo-5,6-dimethoxysalicylamide.

12. [¹²³I] (R)-N- [(1-ethyl-2-pyrrolidinyl)methyl]-5-iodo-2-methoxybenzamide.

13. A radiopharmaceutical composition comprising an effective amount of at least one compound according to any of claims 1 to 12.

## Patentansprüche

1. Verbindung der allgemeinen Formel I 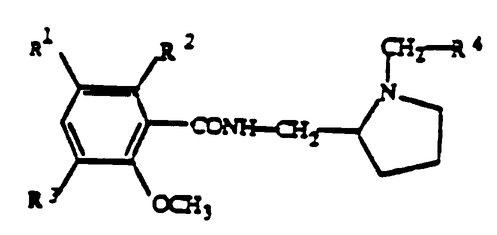 worin R¹ ¹²³I, ¹²⁵I, ¹³¹I ist, R² ein Wasserstoffatom oder eine Hydroxygruppe ist, R³ ein Wasserstoffatom, ein Halogenatom, eine Hydroxy- oder eine Methoxygruppe ist, R⁴ eine Alkylgruppe mit 1 bis 4 Kohlenstoffatomen, eine Alkenylgruppe mit 2 bis 4 Kohlenstoffatomen, eine Alkynylgruppe mit 2 bis 4 Kohlenstoffatomen, eine Cycloalkylgruppe mit 3 bis 7 Kohlenstoffatomen, eine Phenylgruppe, eine para-Halogen-substituierte Phenylgruppe oder ein Enantiomer davon ist, mit der Maßgabe, daß R³ nicht ein Wasserstoffatom ist, wenn R² eine Hydroxygruppe ist.

2. Verbindung nach Anspruch 1, worin R¹ ein Isotop, ausgewählt aus I-123, I-125 und I-131 ist.

3. Verbindung nach Anspruch 1, worin R¹ der Verbindung das I-123 Isotop ist.

4. Verbindung nach Anspruch 1, worin R¹ der Verbindung das I-125 Isotop ist.

5. Verbindung nach Anspruch 1, worin R¹ der Verbindung das I-131 Isotop ist.

6. [¹²³I] (S)-N- [(1-Ethyl-2-pyrrolidinylmethyl]-5-iodo-2-methoxybenzamid.

7. [¹²³I] (R)-N- [(1-(4-Fluorbenzyl)-2-pyrrolidinyl)methyl]-5-iodo-2-methoxybenzamid.

8. [¹²³I] (S)-N- [(1-Ethyl-2-pyrrolidinyl)methyl]-5-chloro-3-iodo-6-methoxysalicylamid.

9. [¹²³I] (R)-N- [(1-(4-Fluorbenzyl)-2-pyrrolidinyl)methyl]-5-iodo-2,3-dimethoxybenzamid.

10. [¹²³I] (S)-N- [(1-Ethyl-2-pyrrolidinyl)methyl]5-iodo-2,3-dimethoxybenzamid.

11. [¹²³I] (S)-N- [(1-Ethyl-2-pyrrolidinyl)methyl]-3-iodo-5,6-dimethoxysalicylamid.

12. [¹²³I] (R)-N- [(1-Ethyl-2-pyrrolidinyl)methyl]-5-iodo-2-methoxybenzamid.

13. Radiopharmazeutische Zusammensetzung, umfassend eine effektive Menge mindestens einer Verbindung nach einem der Ansprüche 1 bis 12.

## Revendications

1. Composé de formule générale I 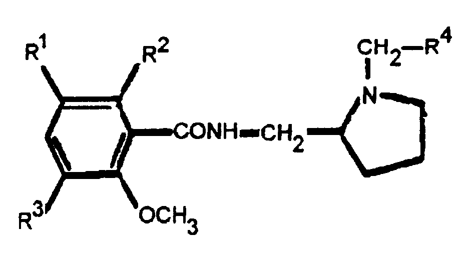 dans laquelle R¹ représente ¹²³I, ¹²⁵I, ¹³¹I, R² représente un atome d'hydrogène ou un groupe hydroxyle, R³ représente un atome d'hydrogène, un atome d'halogène, un groupe hydroxyle, ou un groupe méthoxy, R⁴ représente un groupe alkyle de 1 à 4 atomes de carbone, un groupe alcényle de 2 à 4 atomes de carbone, un groupe alcynyle de 2 à 4 atomes de carbone, un groupe cycloalkyle de 3 à 7 atomes de carbone, un groupe phényle, un groupe phényle substitué par un halogène en position para, ou un énantiomère de celui-ci, à la condition que quand R² représente un groupe hydroxyle, R³ ne représente pas un atome d'hydrogène.

2. Composé selon la revendication 1, dans lequel R¹ est un isotope choisi parmi I-123, I-125 et I-131.

3. Composé selon la revendication 1, dans lequel R¹ du composé est l'isotope I-123.

4. Composé selon la revendication 1, dans lequel R¹ du composé est l'isotope I-125.

5. Composé selon la revendication 1, dans lequel R¹ du composé est l'isotope I-131.

6. ^{[123}I] (S)-N-[(1-éthyl-2-pyrrolidinylméthyl]-5-iodo-2-méthoxybenzamide.

7. [¹²³I](R)-N-[(1-(4-fluorobenzyl)-2-pyrrolidinyl)méthyl]-5-iodo-2-méthoxybenzamide.

8. [¹²³I] (S)-N-[(1-éthyl-2-pyrrolidinyl) méthyl]-5-chloro-3-iodo-6-méthoxysalicylamide.

9. [¹²³I] (R)-N-[(1-(4-fluorobenzyl)-2-pyrrolidinyl)méthyl]-5-iodo-2,3-diméthoxybenzamide.

10. [¹²³I] (S)-N-[(1-éthyl-2-pyrrolidinyl)méthyl]-5-iodo-2,3-diméthoxybenzamide.

11. [¹²³I] (S)-N-[(1-éthyl-2-pyrrolidinyl)méthyl]-3-iodo-5,6-diméthoxysalicylamide.

12. [¹²³I] (R)-N-[(1-éthyl-2-pyrrolidinyl)méthyl]-5-iodo-2-méthoxybenzamide.

13. Composition radiopharmaceutique comprenant une quantité efficace d'au moins un composé selon l'une quelconque des revendications 1 à 12.
